# EUROPÄISCHE PATENTANMELDUNG

(11) **EP 4 101 366 A1**
(43) Veröffentlichungstag der Anmeldung: **14.12.2022**
(21) Anmeldenummer: 22177651.1
(22) Anmeldetag: 07.06.2022
(51) Int. Cl.: A61B 1/12

(54) **VERFAHREN ZUM ERMITTELN EINER VERBINDUNG, VERFAHREN ZUM REINIGEN EINES KANALELEMENTS UND SPÜLGERÄT**

(30) Priorität: 09.06.2021 DE 102021114775
(71) Anmelder: OLYMPUS WINTER & IBE GMBH, 22045 Hamburg (DE)
(72) Erfinder: WERNER, Alexander, 33739 Bielefeld (DE)
(74) Vertreter: Seemann & Partner Patentanwälte mbB

(57) **Zusammenfassung**

Die Erfindung betrifft u.a. ein Verfahren zum Ermitteln einer Verbindung zwischen einem ersten (300) und zumindest einem zweiten (305) in einem Spülgerät (100) befindlichen Kanalelement. Das Verfahren umfasst einen Schritt des Beaufschlagens des ersten Kanalelements (300) mit einem Prüfdruck, einen Schritt des Einlesens eines Drucksignals, das einen Druck in dem zweiten Kanalelement (305) repräsentiert, sowie einen Schritt des Erkennens der Verbindung zwischen dem ersten (300) und dem zumindest zweiten (305) Kanalelement, wenn der Druck in einem vorbestimmten Verhältnis zum Prüfdruck und/oder über einem Schwellwert liegt.

## Beschreibung

Die Erfindung betrifft ein Verfahren zum Ermitteln einer Verbindung, ein Verfahren zum Reinigen eines Kanalelements und ein Spülgerät.

Innerhalb eines Reinigungs- und Desinfektionsgerätes (RDG-E) zur Aufbereitung von Endoskopen kommt es bei pulsierenden Spülverfahren, die auf das Beladungsgut mindestens zwei verschiedene Druckniveaus alternierend beaufschlagen, z. B. Wasser/Luft oder Wasser/Wasser, zu Rückstoßeffekten im Umwälzkreislauf, wenn das Beladungsgut mindestens eine Kanalverzweigung aufweist. Diese Rückstöße können zu vorzeitigen Ausfällen von Komponenten im Spülkreislauf führen, da diese in der Regel nicht für eine solch schlagartige Belastung ausgelegt sind. Beispielsweise könnte eine Umwälzpumpe auf Grund einer Beschädigung zu einem vorzeitigen Ausfall des Gerätes (RDG-E) führen. Eine Vielzahl von Endoskopen verfügt aber über solche Kanalverzweigungen, die zumindest im Biopsiekanal vorkommen. Hier verzweigen sich zwei Kanäle, wovon mindestens ein Kanal am Anschlussstecker des Endoskops mit dem Reinigungs- und Desinfektionsgeräte (RDG-E) verbunden ist und ein weiterer Kanal am Bedienteil mit dem Reinigungs- und Desinfektionsgerät (RDG-E) verbunden wird. Innerhalb des Bedienteils werden die beiden Kanalstücke zusammengeführt und sind als nur noch ein Kanal bis zum distalen Ende ausgeprägt.

Der Erfindung stellt sich als eine Aufgabe, ein verbessertes Verfahren zum Ermitteln einer Verbindung, ein verbessertes Verfahren zum Reinigen eines Kanalelements und ein verbessertes Spülgerät zu schaffen.

Erfindungsgemäß wird diese Aufgabe durch ein Verfahren zum Ermitteln einer Verbindung, ein Verfahren zum Reinigen eines Kanalelements und ein Spülgerät mit den Merkmalen der unabhängigen Ansprüche gelöst. Vorteilhafte Ausgestaltungen und Weiterbildungen der Erfindung ergeben sich aus den nachfolgenden abhängigen Ansprüchen.

Die mit der Erfindung erreichbaren Vorteile bestehen darin, dass in einem Spülgerät eine bestehende fluidische Verbindung zwischen einem ersten und zumindest einem zweiten Kanalelement ermittelt oder erkannt werden kann. Dies ermöglicht eine Erkennung von Kanalverzweigungen in Endoskopkanälen, ohne den Einsatz eines Rückschlagventils.

Die vorliegende Erfindung schafft ein Verfahren zum Ermitteln einer Verbindung zwischen einem ersten und zumindest einem zweiten in einem Spülgerät befindlichen Kanalelement, wobei das Verfahren die folgenden Schritte aufweist:
- Beaufschlagen des ersten Kanalelements mit einem Prüfdruck;
- Einlesen eines Drucksignals, das einen Druck in dem zweiten Kanalelement repräsentiert; und
- Erkennen der Verbindung zwischen dem ersten und dem zumindest zweiten Kanalelement, wenn der Druck in einem vorbestimmten Verhältnis zum Prüfdruck und/oder über einem Schwellwert liegt.

Unter einem Spülgerät kann ein Reinigungs- und Desinfektionsgerät zum Reinigen, Desinfizieren und Aufbereiten von minimalinvasiven, chirurgischen Instrumenten verstanden werden. Bei dem minimalinvasiven chirurgischen Instrument kann es sich beispielsweise um ein Endoskop handeln. Das Endoskop weist beispielhaft zwei Kanalelemente auf: einen Bedienteil und einen Anschlussstecker. Die zwei Kanalelemente des chirurgischen Instruments sind über eine (fluidische) Verbindung miteinander gekoppelt. Bei der (fluidischen) Verbindung kann es sich beispielsweise um einen Schlauch handeln, der ein Fluid zwischen dem ersten und zweiten Kanalelement passieren lassen kann. Zum Prüfen der Verbindung zwischen den beiden Kanalelementen, wird ein erstes Kanalelement mit einem Prüfdruck beaufschlagt. Bei dem Prüfdruck kann es sich beispielsweise um Druckluft mit einem gegenüber einem Umgebungsdruck erhöhten Druck handeln. Anschließend wird der Druck in dem zweiten Kanalelement ermittelt. Wenn der Druck im zweiten Kanalelement dem Prüfdruck entspricht bzw. über einem Schwellenwert des Prüfdrucks liegt, kann eine fluidische Verbindung zwischen den beiden Kanalelementen des chirurgischen Instruments als bestehend angenommen werden.

Der hier vorgestellte Ansatz kann auch als Verfahren zur automatischen Erkennung von Kanalverzweigungen bei englumigen Beladungsgütern (chirurgische Instrumente wie z.B. Endoskope) innerhalb eines Reinigungs- und Desinfektionsgeräts (RDG-E) bezeichnet werden.

Es wird somit ein Verfahren beschrieben, das eine Erkennung von Kanalverzweigungen in Endoskopkanälen bzw. in Kanalelementen von chirurgischen Instrumenten ermöglicht, um so während Entwässerungsroutinen oder Druckluft/Wasser-Taktungen einen Rückstoßeffekt auf die im Spülkreislauf verbaute Pumpe zu verhindern, ohne die Verwendung von Rückschlagventilen im Spülkreislauf zu erfordern, die darüber hinaus aus hygienischer Sicht ebenfalls problematisch sein können.

Der hier vorgestellte Ansatz ermöglicht ferner, dass bei einer Pulsation mit Druckluft während eines Vorspülschrittes nicht alle verzweigten Kanäle gleichzeitig, also parallel mit Druckluft beaufschlagt werden brauchen. Ebenfalls ist nicht in jedem Kanal, der versorgt wird, an gegebener Stelle ein Rückflussverhinderer vorgesehen. Wenn dem Spülgerät keine Informationen vorliegen, welche Kanäle miteinander verbunden, also verzweigt sind, kann das vorliegende System trotzdem erkennen, um welche Kanäle es sich handelt, die eine Verzweigung mit anderen Kanälen aufweisen.

Der hier vorgestellte Ansatz ermöglicht außerdem, dass ein zeitgleiches, paralleles Pulsen aller Kanäle einer Ebene nicht nötig ist, um auszuschließen, dass eine Einzelbeaufschlagung, eine Rückkopplung in den Flottenversorgungszweig und somit einen Druckstoß auf die Umwälzpumpe erfolgt. Die Verwendung von Rückschlagventilen ist dadurch vermeidbar, was bei der Verwendung von herkömmlichen Federrückschlagventilen aus Beständigkeitsgründen im Kontext der eingesetzten Chemikalien (Peressigsäuren treffen auf Federstahl - nicht beständig) problematisch wäre. Bei der Verwendung von Elastomerrückschlagventilen oder zusätzlichen Bypass-Schaltungen wäre neben den nachteiligen hygienischen Eigenschaften zudem ein erheblicher Mehraufwand (Mehrkosten) für die hinzukommenden Komponenten zu berücksichtigen. Vorteilhafterweise wird für den hier vorgestellten Ansatz kein Rückschlagventil verwendet.

Der hier vorgestellte Ansatz kommt ohne die Verwendung von weiteren Hardwarekomponenten, z. B. Rückschlagventilen in Versorgungszweig zwischen Umwälzpumpe und Endoskopüberwachungseinheit, aus und beschreibt das Verfahren (prozessseitig) zur Ermittlung der Kanalverzweigungen. Durch eine initiale Softwareroutine werden in allen nachfolgenden Prozessschritten die verzweigten Kanäle innerhalb eines Endoskops bei einer Pulsation so berücksichtigt, dass die für das Spülgerät effektivste Aufbereitung ohne Rückstoßeffekte im Umwälzkreislauf erfolgen kann.

Das Spülgerät kann unabhängig von dem Vorhandensein einer Endoskopdatenbank erkennen, welche Kanäle des Beladungsgutes (Endoskops) mit anderen Kanälen der Beladung verbunden sind. Zudem gewährt der hier vorgestellte Ansatz ein einwandfreies hygienisches Design für eine Luft/Wasser-Pulsation in einem Spülgerät und kommt ohne Verwendung von Rückschlagventilen im Spülkreislauf aus. Der Integrationsaufwand im Prozess ist zu Beginn des Spülvorganges ohne Verlängerung der Gesamtprozesszeit möglich. Die Prozesssicherheit kann erhöht werden und es besteht keine hinzukommende mechanische Belastung für im Spülkreislauf befindliche Komponenten, wie z. B. Pumpen und/oder die Umwälzpumpe.

Das Verfahren kann gemäß einer Ausführungsform eine Verbindung zwischen dem ersten und zumindest einem dritten in dem Spülgerät befindlichen Kanalelement ermitteln, wobei im Schritt des Einlesens ein weiteres Drucksignal eingelesen werden kann, das einen weiteren Druck in dem dritten Kanalelement repräsentiert, wobei im Schritt des Erkennens die Verbindung zwischen dem ersten und dem zumindest dritten Kanalelement erkannt werden kann, wenn der weitere Druck

in einem vorbestimmten Verhältnis zum Prüfdruck und/oder über einem Schwellwert liegt. Dies bietet den Vorteil, dass eine weitere Verbindung zu einem dritten Kanalelement im Spülgerät einfach und effizient geprüft und/oder erkannt werden kann.

Das Verfahren kann gemäß einer Ausführungsform eine Verbindung zwischen dem zweiten und zumindest dem dritten in dem Spülgerät befindlichen Kanalelement ermitteln, wobei im Schritt des Beaufschlagens das zweite Kanalelement mit einem zweiten Prüfdruck beaufschlagt werden kann, wobei im Schritt des Erkennens die Verbindung zwischen dem zweiten und dem zumindest dritten Kanalelement erkannt werden kann, wenn der weitere Druck in einem vorbestimmten Verhältnis zum weiteren Prüfdruck und/oder über einem weiteren Schwellwert liegt. Dies bietet den Vorteil, dass eine Verbindung zwischen dem zweiten und zumindest dem dritten Kanalelement einfach und effizient geprüft und/oder erkannt werden kann.

Gemäß einer Ausführungsform kann vor dem Schritt des Beaufschlagens des zweiten Kanalelements mit dem zweiten Prüfdruck der Prüfdruck aus dem ersten Kanalelement abgelassen und/oder zumindest reduziert werden. Dies kann die Prozesssicherheit erhöhen und eine Erkennung einer fluidischen Verbindung verbessern.

Das Verfahren kann gemäß einer Ausführungsform einen Schritt des Speicherns einer Information über das Vorhandensein der Verbindung in einer Tabelle aufweisen, insbesondere wobei die Zeilen und die Spalten je eines der Kanalelemente repräsentieren. Dies bietet den Vorteil, dass das Spülgerät mit einfachen Mitteln erkennen kann, welche Kanalelemente mit anderen Kanalelementen verbunden sind.

Gemäß einer Ausführungsform kann im Schritt des Beaufschlagens Druckluft und/oder eine Flüssigkeit in das erste Kanalelement gepumpt werden, um den Prüfdruck aufzubauen. Speziell bei der Verwendung einer Reinigungsflotte kann mit einer solchen Vorgehensweise schnell und einfache die fluidische Verbindung erkannt werden wobei in diesem Fall bereits eine erste Reinigung der Kanalelemente ausgeführt werden kann und die Reinigung dann auch schneller abgeschlossen werden kann.

Gemäß einer weiteren Ausführungsform kann im Schritt des Beaufschlagens der Prüfdruck in ein medizinisches Gerät als Kanalelement, insbesondere in ein Endoskopelement beaufschlagt werden und/oder wobei im Schritt des Einlesens ein Druck in einem zweiten medizinischen Gerät als zweiten Kanalelement, insbesondere in einem zweiten Endoskopelement eingelesen werden kann. Dies bietet den Vorteil, dass Endoskopelemente optimal und mit geringem Aufwand gereinigt oder aufbereitet werden können.

Ferner kann gemäß einer Ausführungsform im Schritt des Beaufschlagens das Kanalelement unter Verwendung eines 3/2-WegeVentils mit dem Prüfdruck beaufschlagt werden. Dies bietet den Vorteil, dass der Weg des Volumenstroms optimal und mit technisch einfachen Mitteln gesteuert werden kann.

Im Schritt des Beaufschlagens kann gemäß einer Ausführungsform das Kanalelement durch einen Druckanschluss eines Pulsationsmoduls mit dem Prüfdruck beaufschlagt werden und wobei im Schritt des Einlesens das Drucksignal von einem Drucksensor des Pulsationsmoduls eingelesen werden kann, insbesondere wobei der Drucksensor und der Druckanschluss in einem gemeinsamen Pulsationsmodulgehäuse angeordnet sind. Dies kann die Prozesssicherheit erhöhen.

Die Schritte des Beaufschlagens, des Einlesens und/oder des Erkennens können gemäß einer Ausführungsform ausgeführt werden, wenn eine Umwälzpumpe zur Umwälzung einer Behandlungsflotte eingeschaltet oder ausgeschaltet ist. Dies bietet den Vorteil, dass das Verfahren unabhängig von der Umwälzpumpe durchgeführt werden kann und somit keine zusätzliche mechanische Belastung für die im Spülkreislauf befindlichen Komponenten, wie die Umwälzpumpe entsteht. Alternativ kann auch während eines Umwälzens das Erkennen erfolgen, sodass eine schnellere Reinigung der Kanalelemente erreicht werden kann.

Besonders günstig ist es gemäß einer Ausführungsform auch ein Verfahren zum Reinigen eines Kanalelements vorzusehen, wobei das Verfahren einen Schritt des Durchleitens einer Behandlungsflotte durch das erste und/oder zweite Kanalelement umfasst, ansprechend auf die in dem genannten Verfahren erkannte Verbindung. Dies bietet den Vorteil, dass das Kanalelement hygienisch aufbereitet werden kann. Zudem kann die Aufbereitung ohne den Einsatz eines Rückschlagventils durchgeführt werden.

Eine Vorrichtung kann die Schritte eines der Verfahren in entsprechenden Einheiten ausführen und/oder ansteuern.

Ein Spülgerät kann gemäß einer Ausführungsform die Vorrichtung, eine Spülkammer und zumindest einen Spülkorb aufweisen.

Der hier vorgestellte Ansatz schafft ferner eine Vorrichtung, die ausgebildet ist, um die Schritte einer Variante eines der hier vorgestellten Verfahren in entsprechenden Einrichtungen durchzuführen, anzusteuern bzw. umzusetzen. Auch durch diese Ausführungsvariante der Erfindung in Form einer Vorrichtung kann die der Erfindung zugrunde liegende Aufgabe schnell und effizient gelöst werden.

Die Vorrichtung kann ausgebildet sein, um Eingangssignale einzulesen und unter Verwendung der Eingangssignale Ausgangssignale zu bestimmen und bereitzustellen. Ein Eingangssignal kann beispielsweise ein über eine Eingangsschnittstelle der Vorrichtung einlesbares Sensorsignal darstellen. Ein Ausgangssignal kann ein Steuersignal oder ein Datensignal darstellen, das an einer Ausgangsschnittstelle der Vorrichtung bereitgestellt werden kann. Die Vorrichtung kann ausgebildet sein, um die Ausgangssignale unter Verwendung einer in Hardware oder Software umgesetzten Verarbeitungsvorschrift zu bestimmen. Beispielsweise kann die Vorrichtung dazu eine Logikschaltung, einen integrierten Schaltkreis oder ein Softwaremodul umfassen und beispielsweise als ein diskretes Bauelement realisiert sein oder von einem diskreten Bauelement umfasst sein.

Von Vorteil ist auch ein Computerprogrammprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann. Wird das Programmprodukt oder Programm auf einem Computer oder einer Vorrichtung ausgeführt, so kann das Programmprodukt oder Programm zur Durchführung, Umsetzung und/oder Ansteuerung der Schritte der Verfahren nach einer der vorstehend beschriebenen Ausführungsformen verwendet werden.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen. Im Rahmen der Erfindung sind Merkmale, die mit "insbesondere" oder "vorzugsweise" gekennzeichnet sind, als fakultative Merkmale zu verstehen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird.

Ein Ausführungsbeispiel der Erfindung ist in den Zeichnungen rein schematisch dargestellt und wird nachfolgend näher beschrieben. Es zeigt
- Figur 1: eine Darstellung eines Ausführungsbeispiels eines Spülgerätes;
- Figur 2: eine Darstellung eines Pulsationsmoduls für ein Ausführungsbeispiel eines Spülgerätes;
- Figur 3: eine Darstellung eines Reinigungsgutes zur Verwendung in einem Ausführungsbeispiel eines Spülgerätes;
- Figur 4: eine tabellarische Übersicht von Verbindungen zwischen Kanalelementen zur Verwendung in einem Ausführungsbeispiel eines Spülgerätes;
- Figur 5: eine tabellarische Übersicht von Druckniveaus relevanter Komponenten Ausführungsbeispiels eines eines Spülgerätes;
- Figur 6: ein Struktogramm eines Ausführungsbeispiels eines Verfahrens zum Ermitteln einer Verbindung;
- Figur 7: eine Ausgabematrix eines Ausführungsbeispiels eines Verfahrens zum Ermitteln einer Verbindung;
- Figur 8: ein Blockschaltbild einer Vorrichtung gemäß einem Ausführungsbeispiel;
- Figur 9: ein Ablaufdiagramm eines Ausführungsbeispiels eines Verfahrens zum Ermitteln einer Verbindung zwischen einem ersten und zumindest einem zweiten in einem Spülgerät befindlichen Kanalelement; und
- Figur 10: ein Ablaufdiagramm eines Ausführungsbeispiels eines Verfahrens zum Reinigen eines Kanalelements.

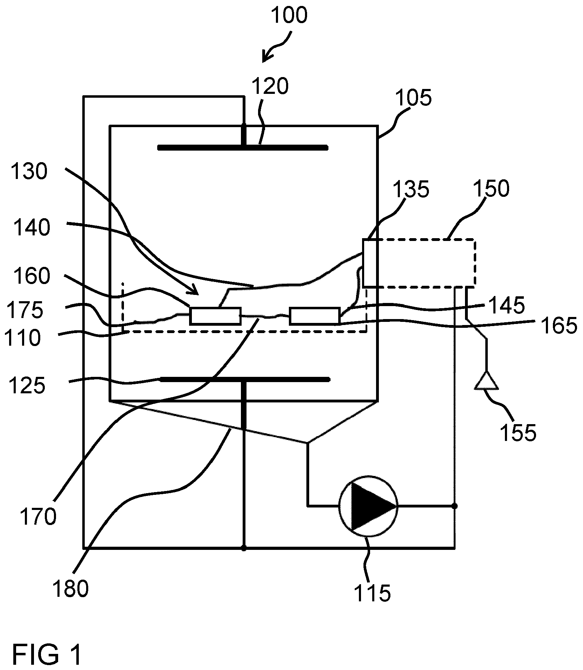

Figur 1 zeigt eine Darstellung eines Ausführungsbeispiels eines Spülgerätes 100 gemäß einem Ausführungsbeispiel. Bei dem Spülgerät 100 kann es sich um ein Reinigungs- und Desinfektionsgerät zum Reinigen, Desinfizieren und Aufbereiten von minimalinvasiven, chirurgischen Instrumenten, wie z.B. Endoskope oder dergleichen handeln. Das Spülgerät 100 weist eine Spülkammer 105 auf. Die Spülkammer 105 ist beispielhaft zur Aufnahme eines Spülkorbes 110 ausgebildet. Beispielhaft befindet sich unterhalb des Spülkorbes 110 eine Umwälzpumpe 115, die ausgebildet ist, um eine Flüssigkeit, hier eine Behandlungsflotte, über Leitungen in die Spülkammer 105 zu dem Spülkorb 110 zu pumpen.

Optional weist das Spülgerät 100 zwei Sprüharme 120, 125 auf, über die die Behandlungsflotte in die Spülkammer 105 gesprüht wird. Dabei befindet sich beispielhaft ein erster Sprüharm 120 an einer Decke der Spülkammer 105 oberhalb des Spülkorbes 110 und ein zweiter Sprüharm 125 an einem Boden der Spülkammer 105 unterhalb des Spülkorbes 110.

Der Spülkorb 105 ist vorgesehen, um Reinigungsgut 130 gemäß einem Ausführungsbeispiel medizinisches Gerät zum Reinigen und zusätzlich oder alternativ zum Desinfizieren aufzunehmen. In Figur 1 ist schematisch ein sich in dem Spülkorb 105 angeordnetes Reinigungsgut 130 in Form eines Endoskops dargestellt. Zum Anbinden des Reinigungsgutes 130 an das Spülgerät 100 weist der Spülkorb 110 eine Adaptereinrichtung 135 auf. Beispielsweise ist die Adaptereinrichtung 135 als Adapterplatte ausgeführt und an einer Wand des Spülkorbes 110 befestigt. Das Reinigungsgut 130 ist beispielhaft mit zwei Schläuchen 140,145 an der entsprechenden Adaptereinrichtung 135 befestigt. Dazu weist die Adaptereinrichtung 135 Anschlussstutzen auf, über die die Schläuche 140,145 angeschlossen werden können.

Um das Reinigungsgut 130 über die Adaptereinrichtung 135 an das Spülgerät 100 anzubinden ist die Adaptereinrichtung 135 an einem Pulsationsmodul 150 angekoppelt. In diesem Zustand kann die Behandlungsflotte durch das Pulsationsmodul 150 hindurch zu der Adaptereinrichtung 135 und von der Adaptereinrichtung 135 zu dem Reinigungsgut 130 geleitet werden. Ferner weist das Spülgerät 100 eine Druckluftversorgung 155 auf, die ausgebildet ist, um Druckluft in das Reinigungsgut 130 zu leiten. Die Druckluft wird, ähnlich wie die Behandlungsflotte, über das Pulsationsmodul 150 zu dem Reinigungsgut 130 geleitet. Die Schläuche 140, 145 sind dabei ausgebildet, um die Behandlungsflotte und die Druckluft durch das Pulsationsmodul 150 hindurch zu der Adaptereinrichtung 135 und von der Adaptereinrichtung 135 zu dem Reinigungsgut 130 zu leiten.

Das Reinigungsgut 130 umfasst beispielhaft ein Bedienteil 160 und einen Anschlussstecker 165, wobei das Bedienteil 160 und der Anschlussstecker 165 über ein Verbindungselement 170 miteinander verbunden sind. Dabei ist das Bedienteil 160 mit dem Schlauch 140 an die Adaptereinrichtung 135 gekoppelt, während der Anschlussstecker 165 mit dem Schlauch 145 an die Adaptereinrichtung 135 gekoppelt ist. Das Bedienteil 160 weist beispielhaft ein distales Ende 175 auf. Unterhalb der Spülkammer 105 befindet sich beispielhaft ein Pumpensumpf 180, der ausgebildet ist, um die Umwälzpumpe 115 mit der Behandlungsflotte zu speisen. Vor einem Reinigungs- und/oder Spülvorgang wird beispielhaft ermittelt, ob eine Verbindung zwischen dem Anschlussstecker 165 und dem Bedienelement 160 besteht.

Dazu wird beispielhaft der Anschlussstecker 165, der als ein erstes Kanalelement 165 ausgeformt ist, mit einem Prüfdruck beaufschlagt. Der Prüfdruck wird aus der Druckluftversorgung 155 durch das Pulsationsmodul 150 über den Schlauch 145 zu dem ersten Kanalelement 165 geleitet. Anschließend wird ein Drucksignal eingelesen, dass einen Druck in dem Bedienelement 160, das als ein zweites Kanalelement 160 ausgeformt ist, repräsentiert. Wenn der Druck in einem vorbestimmten Verhältnis zu dem Prüfdruck steht und/oder über einem Schwellwert liegt, wird eine Verbindung zwischen dem ersten 165 und dem zweiten Kanalelement 160 erkannt.

Das beschriebene Verfahren wird innerhalb eines Spülgerätes 100 eingesetzt, das auch als Reinigungs- und Desinfektionsgerätes (RDG-E) bezeichnet werden kann, das über eine Spülkammer 105, die auch als Spülraum bezeichnet werden kann, einen Pumpensumpf 180 der eine Umwälzpumpe 115 speist, die sowohl den ersten Sprüharm 120, der auch als oberer Spülarm bezeichnet werden kann, als auch den zweiten Sprüharm 125, der auch als unterer Spülarm bezeichnet werden kann versorgt. Zudem ist der Ausgang der Umwälzpumpe 115 durch eine Flotten-Verbindung mit einem Pulsationsmodul 150 verbunden. Das Pulsationsmodul 150 weist zudem mindestens eine Druckluftversorgung 155 auf, die über ein höheres, im Vergleich zur Flotten-Verbindung, Druckniveau verfügt. Das Reinigungsgut 130, das auch als Beladungsgut bezeichnet werden kann, also das Endoskop, bestehend aus einem Anschlussstecker 165, einem Bedienteil 160, einer Verbindung 170, die den Anschlussstecker 165 mit dem Bedienteil 160 verbindet, einem Endoskopschlauch, der am distalen Ende 175 endet, sowie einem Schlauch 135, der auch als Verbindung bezeichnet werden kann vom Bedienteil 160 zur Adaptereinrichtung 135 als auch ein Schlauch 145, der auch als Verbindung bezeichnet werden kann vom Anschlussstecker 165 zur Adaptereinrichtung 135, wobei die beiden Schläuche 140, 145 zur Adaptereinrichtung 135 dazu genutzt werden, dass Endoskop mit dem Spülgerät 100 zu verbinden.

Der Aufbereitungsprozess innerhalb des Spülgerätes 100 besteht beispielsweise aus den nachfolgenden Prozessschritten:
- Dichtigkeitstest durchführen und Überwachung für Folgeschritte starten
- Vorspülphase (Wassereinlauf, Spülen, Wasserablauf)
- Reinigungsphase (Wassereinlauf und Dosierung Reinigungschemie und Heizen, Spülen, Wasserablauf)
- Zwischenspülen (Wassereinlauf, Spülen, Wasserablauf)
- Desinfektionsphase (Wassereinlauf und Dosierung Desinfektionsmittel und Heizen, Spülen, Wasserablauf)
- Nachspülen 1 (Wassereinlauf, Spülen, Wasserablauf)
- Nachspülen 2 (Wassereinlauf und Heizen falls Trocknung folgt, Spülen, Wasserablauf)
- Abschluss-Entwässern oder Trocknen

Das beschriebene Verfahren wird während der Aufbereitung im Reinigungsgerät 100 beispielhaft nur einmal, möglichst zu Beginn der Aufbereitung ausgeführt, z. B. parallel zur Dichtigkeitstestdurchführung und Überwachung für Folgeschritte oder während der Vorspülphase.

Die beschriebenen Informationen werden beispielsweise genutzt, um eine effektivere Entwässerung zwischen den Spülphasen, während der Abschluss-Entwässerung oder während der Trocknung der inneren Kanäle durchzuführen. Es ist somit möglich, auch Fremdendoskope vom Spülgerät 100 so aufzubereiten, dass eine an den vorliegenden Endoskoptyp angepasste, optimierte Trocknung oder Entwässerung stattfindet. Gleiches gilt für die Durchflussmessung. Verzweigte Kanäle von Fremdendoskopen oder Endoskopen, von denen die Verzweigungsinformationen initial nicht bekannt sind, werden während der Durchflussmessung eines verzweigten Kanals parallel gemessen.

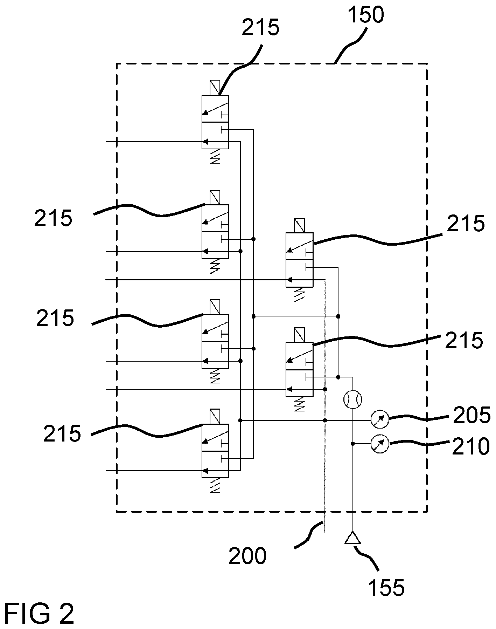

Figur 2 zeigt eine Darstellung eines Pulsationsmoduls 150 für ein Ausführungsbeispiel eines Spülgerätes. Bei dem Pulsationsmodul 150 kann es sich um das in Figur 1 beschriebene Pulsationsmodul 150 handeln. Das Pulsationsmodul 150 ist beispielhaft in einem Spülgerät verbaut. Unterhalb des Pulsationsmoduls 150 ist beispielhaft eine Druckluftversorgung 155 angeordnet, die die Ventile 215 mit Druckluft bzw. Prüfdruck beaufschlägt. Durch die Ventile 215 fließt beispielhaft die Behandlungsflotte, die über eine Flotten-Verbindung 200 erfolgt. In dem Pulsationsmodul 150 sind beispielhaft zwei Drucksensoren 205, 210 angeordnet, wobei der eine Drucksensor 205 als Flotten-Drucksensor ausgebildet ist und der zweite Drucksensor 210 als Druckluftsensor ausgebildet ist.

Das Pulsationsmodul 150 besteht aus einer Anzahl von 3/2-WegeVentilen 215, die in der einen Schaltstellung dafür sorgen, dass ein Endoskopkanal mit Behandlungsflotte, die auch als Spülflotte bezeichnet werden kann, durch die Umwälzpumpe versorgt werden. In der anderen Schaltstellung wird der jeweilige Endoskopkanal mit Druckluft beaufschlagt und die Verbindung zur Flottenversorgung unterbrochen. Sowohl der Drucklufteingang, als auch die Flottenversorgung des Pulsationsmoduls 150 sind mit einem Drucksensor 205, 210 verbunden.

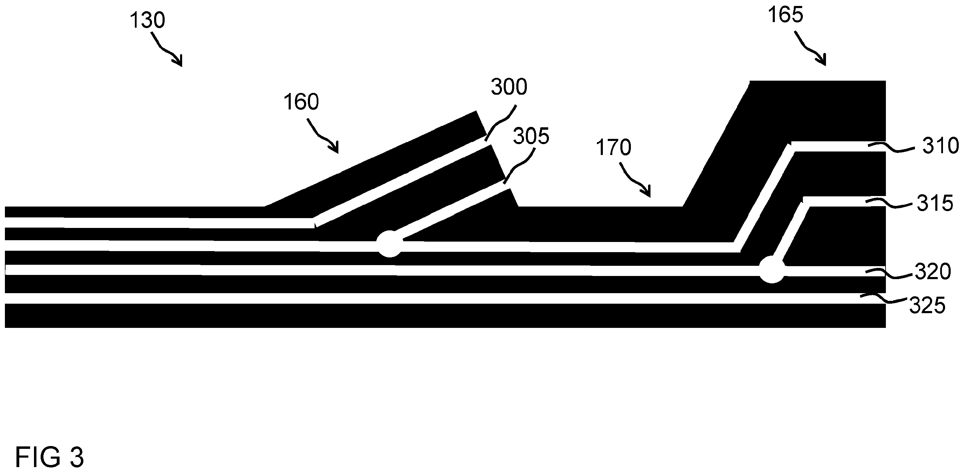

Figur 3 zeigt eine Darstellung eines Reinigungsgutes 130 zur Verwendung in einem Ausführungsbeispiel eines Spülgerätes 100.

Bei dem Reinigungsgut 130 kann es sich um das in Figur 1 beschriebene Reinigungsgut 130 handeln. Das Reinigungsgut 130 ist in Figur 3 beispielhaft als Endoskop ausgeformt. Figur 3 zeigt, welche Kanäle mit welchen anderen Kanälen verbunden sind. Das Reinigungsgut 130 besteht beispielhaft aus dem Bedienelement 160 und dem Anschlussstecker 165.

Das Bedienelement 160 weist beispielhaft zwei Kanalelemente 300, 305 auf und ist über die Verbindung 170 mit dem Anschlussstecker 165 gekoppelt. Der Anschlussstecker 165 weist beispielhaft vier Kanalelemente 310, 315, 320, 325 auf.

Gemäß einem Ausführungsbeispiel ist das Kanalelement 305 des Bedienelementes 160 mit dem Kanalelement 310 des Anschlusssteckers verbunden. Das Kanalelement 315 ist mit dem Kanalelement 320 verbunden.

Ein Ablaufbeispiel eines Algorithmus unter Verwendung des in Figur 2 gezeigten Pulsationsmoduls verläuft beispielhaft folgendermaßen: Die Pumpe im Spülgerät läuft während der Überprüfung der Verbindungen der Kanalelemente mit reduzierter Drehzahl in einem Solldruckbereich von z. B. DSF=450...550 mBar. Das Druckluftniveau beträgt beispielsweise DSL=1450 bis 1550 mBar.

Nun wird eine Laufvariable i=1 gesetzt und ein Grenzwert bzw. eine Toleranz der Messwerte festgelegt, z. B. Tol =100 mBar. Anschließend wird das erste Kanalelement 300 auf eine Verbindung mit einem weiteren Kanalelement hin überprüft. Dazu wird ein Ventil V1 in dem ersten Kanalelement 300 ein- bzw. durchgeschaltet (i=1). Es wird geprüft, ob im ersten Kanalelement 300 ein erhöhter Druck an einem Drucksensor messbar ist. Dies ist nicht der Fall, was bedeutet, dass das erste Kanalelement 300 keine Verbindung zu einem weiteren Kanalelement aufweist. Nachdem das erste Kanalelement 300 auf eine Verbindung hin überprüft wurde, wird das Ventil V1 des ersten Kanalelementes 300 wieder ausgeschaltet. Nun wird i=i+1 gesetzt und das Ventil V2 des zweiten Kanalelementes 305 eingeschaltet. Es wird geprüft, ob im zweiten Kanalelement 305 ein Druck an dem Drucksensor messbar ist. In diesem Fall ist ein Druck messbar, sodass geschlussfolgert wird, dass das zweite Kanalelement 305 eine (fluidische) Verbindung mit einem weiteren Kanalelement aufweist. Anschließend wird geprüft, mit welchem Kanalelement das zweite Kanalelement 305 verbunden ist. Dazu wird eine zweite Laufvariable j=1 gesetzt und das Ventil V1 des ersten Kanalelementes 300 eingeschaltet. Nun wird geprüft, ob sich der Druck im Drucksensor wieder auf seinen Sollwert, beispielsweise 450...550 mBar, normalisiert. Dies ist nicht Fall. Das zweite Kanalelement 305 ist also nicht mit dem ersten Kanalelement 300 verbunden. Daher wird das Ventil V1 des ersten Kanalelementes 300 ausgeschaltet, da i ungleich j ist. Nun wird j=j+1 (j=2) gesetzt und das Ventil V2 des zweiten Kanalelementes 305 eingeschaltet, bzw. war es schon vorher eingeschaltet. Anschließend wird auch hier geprüft, ob sich der Druck im Drucksensor wieder auf seinen Sollwert, 450...550 mBar, normalisiert. Dies ist nicht der Fall.

Das zweite Kanalelement 305 ist nicht nur mit dem zweiten Kanalelement 305 (fluidisch) verbunden. Das Ventil V2 des zweiten Kanalelementes 305 wird ausgeschaltet, wenn i ungleich j ist. Da i gleich j ist, bleibt das Ventil V2 des zweiten Kanalelementes 305 angeschaltet. Nun wird j=j+1 (j=3) gesetzt und das dritte Kanalelement 310 eingeschaltet. Anschließend wird auch hier geprüft, ob sich der Druck im Drucksensor wieder auf seinen Sollwert, 450...550mBar, normalisiert. Der Druck normalisiert sich wieder, sodass das zweite Kanalelement 305 des Bedienelementes 160 mit dem dritten Kanalelement 310 des Anschlusssteckers 165 verbunden ist. Nun wird eine Matrix KV(2;3)=1 gesetzt. Die Matrix bedeutet, dass eine Verbindung zwischen dem zweiten Kanalelement 305 und dem dritten Kanalelement 310 besteht. Das Ventil V3 des dritten Kanalelementes 310 wird anschließend ausgeschaltet, da i ungleich j ist. Nun wird j=j+1 (j=4) gesetzt und das vierte Kanalelement 315 eingeschaltet. Die Verbindungen der Kanalelemente 315, 320 und 325 werden solange geprüft (i=2) bis j=6 ist. Anschließend wird das Ventil V2 des zweiten Kanalelementes 305 ausgeschaltet und i=i+1 gesetzt und das Ventil V3 des dritten Kanalelementes 310 eingeschaltet. Es wird nun geprüft, ob ein erhöhter Druck am Drucksensor messbar ist. Dies ist der Fall, sodass geschlussfolgert wird, dass das zweite Kanalelement 305 eine (fluidische) Verbindung mit einem weiteren Kanalelement aufweist. Dies ist schon von den vorherigen Überprüfungen bekannt.

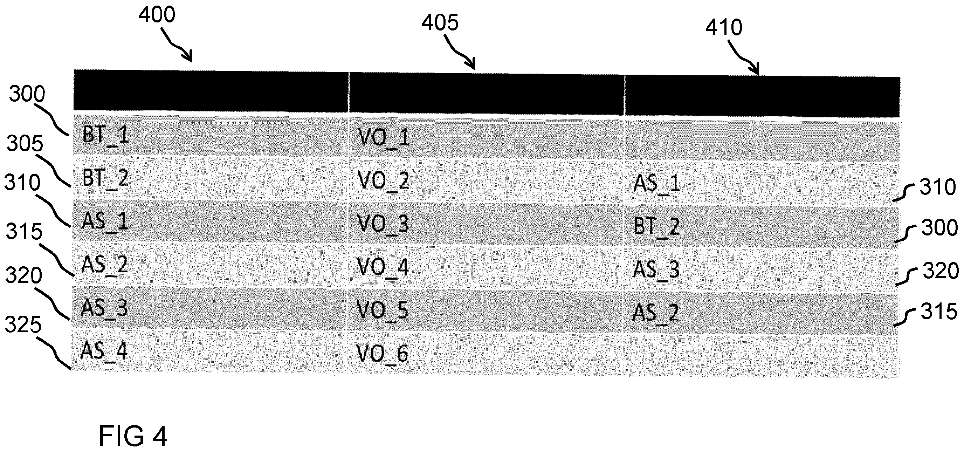

Figur 4 zeigt eine tabellarische Übersicht von Verbindungen zwischen Kanalelementen zur Verwendung in einem Ausführungsbeispiel eines Spülgerätes 100. Dabei handelt es sich um eine Anschlusstabelle der Verbindungen des Reinigungsgutes aus Figur 3. In der linken Spalte 400 sind die Kanalelemente aufgelistet. In der rechten Spalte 410 sind die Kanalelemente aufgelistet, die mit den Kanalelementen aus der linken Spalte 400 verbunden sind. In der mittleren Spalte 405 sind die Verbindungen aufgelistet, über die einzelne Kanalelemente miteinander verbunden sind. Beispielhaft weist das erste Kanalelement 300 eine Verbindung auf, ist jedoch an kein weiteres Kanalelement angeschlossen. Das zweite Kanalelement 305 weist eine Verbindung auf und ist über diese Verbindung mit dem dritten Kanalelement 310 verzweigt. Das dritte Kanalelement 310 ist wiederum über eine Verbindung mit dem zweiten Kanalelement 305 verzweigt. Das vierte Kanalelement 315 ist über eine Verbindung mit dem fünften Kanalelement 320 verzweigt. Das fünfte Kanalelement 320 ist wiederum über eine Verbindung mit dem vierten Kanalelement 315 verzweigt. Das sechste Kanalelement 325 weist eine Verbindung auf, ist aber mit keinem Kanalelement verzweigt.

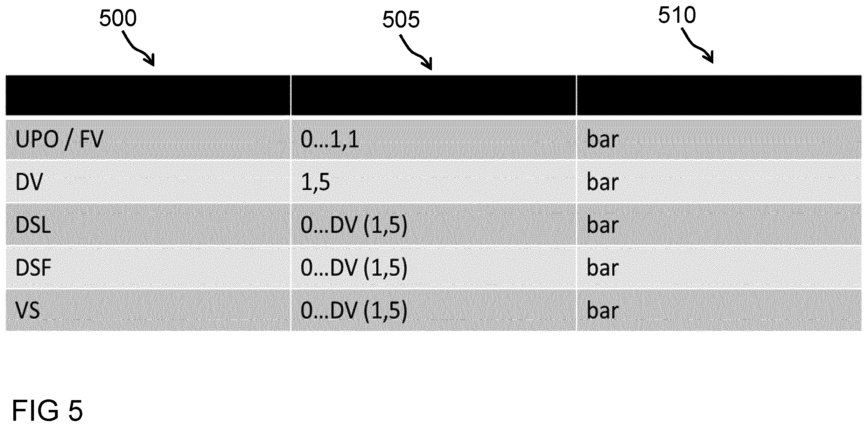

Figur 5 zeigt eine tabellarische Übersicht von Druckniveaus relevanter Komponenten eines Ausführungsbeispiels eines Spülgerätes 100. In der linken Spalte 500 sind die Komponenten, in der mittleren Spalte 505 die verschiedenen Druckniveaus aufgelistet und in der rechten Spalte 510 ist die Druckeinheit angegeben. Der Ausgang der Umwälzpumpe 115 bzw. die Flotten-Verbindung 200 weist ein Druckniveau von beispielsweise 0...1,1 bar auf. Die Druckluftversorgung 155 weist ein Druckniveau von beispielsweise 1,5 bar. Der Druckluftsensor 210, der Flotten-Drucksensor 205 und der Volumenstrom 515 weisen jeweils ein Druckniveau von beispielsweise 1,5 bar auf.

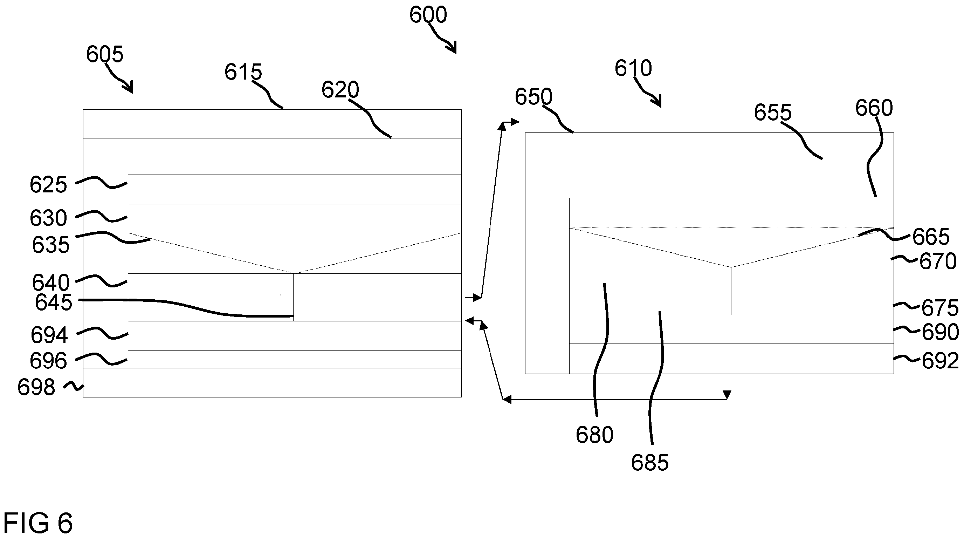

Figur 6 zeigt ein Struktogramms 600 eines Ausführungsbeispiels eines Verfahrens zum Ermitteln einer Verbindung. Das Struktogramm 600 weist einen linken Block 605 und einen rechten Block 610 auf. In dem linken Block 605 werden die Kanalverzweigungen erkannt und in dem rechten Block 610 die Kanalverzweigungen überprüft. Das Verfahren startet, indem eine Anweisung 615 erfolgt, die i=1 setzt und den Toleranzbereich des Druckes festlegt, beispielsweise 100 mBar. Anschließend wird die Anweisung 620 festgelegt, die besagt, dass die nachfolgenden Anweisungen solange durchgeführt bzw. wiederholt werden, wie i<=6 gilt. Als nächstes folgt die Anweisung 625, in der der Flotten-Drucksensor_i =Flotten-Drucksensor; gesetzt wird. Wenn die Umwälzpumpe ausgeschaltet ist, dann gilt: Flotten-Drucksensor_i =0. In der Anweisung 630 gilt Vi=1, das Ventil Vi des ersten Kanalelementes wird eingeschaltet. Anschließend folgt die Bedingung 635, in der geprüft wird, ob der Flotten-Drucksensor größer als der Flotten-Drucksensor_i plus Toleranzbereich ist. Trifft die Bedingung nicht zu, wird in der Anweisung 640 ermittelt, dass das erste Kanalelement keine Verzweigung zu einem anderen Kanalelement aufweist. Trifft die Bedingung 635 zu, erfolgt der Funktionsaufruf die Kanalverzweigungen zu überprüfen und es wird zu Block 610 gesprungen, in dem die Kanalverzweigungen überprüft werden. Dazu wird die Anweisung 650 j=1 gesetzt, gefolgt von der Anweisung 655, dass die nachfolgenden Anweisungen solange durchgeführt bzw. wiederholt werden, wie j<=6 gilt. Anschließend folgt die Anweisung 660 in der das Ventil Vj des zweiten Kanalelementes eingeschaltet wird, das Ventil Vi des ersten Kanalelementes ist bereits aktiv.

Anschließend folgt die Bedingung 665, in der geprüft wird, ob der Flotten-Drucksensor kleiner als der Flotten-Drucksensor_i plus Toleranzbereich ist. Trifft die Bedingung nicht zu, wird in der Anweisung 670 keine Kanalverzweigung ermittelt. Die entsprechende Matrix 675 lautet: Matrix_KV(i:j)=0. Trifft die Bedingung 665 zu, wird in der Anweisung 680 eine Verzweigung zwischen dem ersten Kanalelement und dem zweiten Kanalelement erkannt. Die entsprechende Matrix 685 lautet: Matrix_KV(i:j)=1. In der Anweisung 690 heißt es, dass wenn i nicht gleich j ist, ist Vj=0. Daraufhin wird das Ventil Vj des zweiten Kanalelementes ausgeschaltet. In der Anweisung 692 wird j=j+1 gesetzt. Anschließend wird wieder zu Block 605 gesprungen. In Block 605 wird in der Anweisung 694 Vi=0 gesetzt und somit das Ventil Vi des ersten Kanalelementes ausgeschaltet. In Block 696 wird i=i+1 gesetzt und abschließend in der Anweisung 698 die Matrix Matrix_KV(6x6) ausgegeben und das Verfahren zum Ermitteln einer Verbindung zwischen einem ersten und zumindest einem zweiten in einem Spülgerät befindlichen Kanalelement abgeschlossen.

Das Verfahren kann durchgeführt werden, wenn die Umwälzpumpe eingeschaltet oder ausgeschaltet ist. Die übliche Auslegung innerhalb eines Spülgerätes (RDG-E) für die Durchführung des Verfahrens sieht folgendermaßen aus: Der Pumpendruck ist kleiner als der Druck der Druckluftversorgung. Das Verfahren prüft jeden Kanal nacheinander, solange in der Anweisung 620 die i<=6 Schleife gilt, ob eine Verbindung zu einem anderen Kanal besteht, wenn der Flotten-Drucksensor (DSF) einen höheren Druck als seinen üblichen Druckwert besitzt, wenn die Umwälzpumpe ein oder ausgeschaltet ist. Dies erfolgt durch die Prüfung 635 DSF>=DSF_i + Tol.

Ist in der Anweisung 635 bei geschaltetem Ventil Vi innerhalb der ersten Schleife diese Rückkopplung vorhanden, durch Erfüllung der vorherigen Prüfbedingung, so wird nun geprüft, mit welchem Kanal der aktive Kanal verbunden ist. Dies erfolgt über eine weitere, untergeordnete Schleife (Anweisung 655, Solange j<=6), die nach dem Funktionsaufruf Verzweigung_prüfen(i) durchlaufen wird. Hier wird in der Anweisung 660 zum aktuell aktiven Ventil Vi nacheinander stets ein weiteres Ventil j zugeschaltet. Immer dann, wenn bei zwei parallel aktiven Ventilen die Prüfbedingung DSF <=DSF_i + Tol erfüllt wird, also der Druck in der Flottenversorgung sich wieder normalisiert, kann davon ausgegangen werden, dass der aktuelle Kanal i mit dem Kanal j verbunden sind. Um dies zu dokumentieren, wird in einer Ausgabematrix Matrix_KV(i,j) an der jeweiligen Stelle eine "1" gesetzt-> Kanal i ist mit Kanal j verbunden. An allen anderen Stellen, mit dem Kanal i keine Verbindung zu anderen Kanälen (j) aufweist, wird eine 0 gesetzt.

Anhand der Matrix_KV(i,j) 675 welche die Dimension n × n (in unserem Beispiel also 6 × 6) besitzt, kann das Reinigungs- und Desinfektionsgerät (RDG-E) nun in allen nachfolgenden Entwässerungsroutinen die miteinander Verbundenen Kanäle stets parallel mit Druckluft beaufschlagen, sodass es entgegen einer sequentiellen Einzelkanalbeaufschlagung mit Druckluft in einem späteren Entwässerungsschritt nun nicht mehr zu Rückstoßeffekten über eine Kanalverzweigung (z. B. Figur 3, das zweite Kanalelement ist mit dem dritten Kanalelement verbunden) auf die Flottenversorgung und somit die Umwälzpumpe kommt.

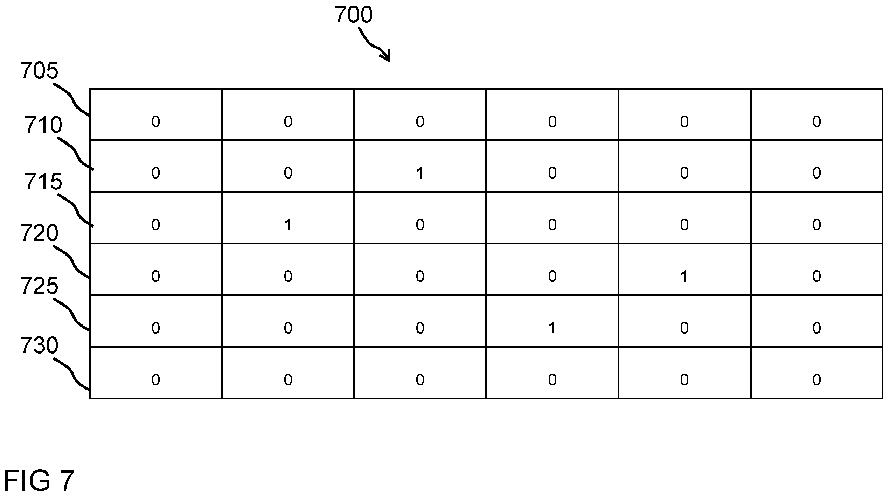

Figur 7 zeigt eine Ausgabematrix eines Ausführungsbeispiels eines Verfahrens zum Ermitteln einer Verbindung. Dabei kann es sich um die in Figur 3, Figur 4 und Figur 6 beschriebenen Verbindungen zwischen einzelnen Kanalelementen handeln.

Die Ausgabematrix lautet Matrix_KV(6x6) und ist in Figur 7 tabellarisch dargestellt. Für die Zeile 705 gilt: 0 0 0 0 0 0. Dies bedeutet, dass das erste Kanalelement keine Kanalverzweigung aufweist. Für die Zeile 710 gilt: 0 0 1 0 0 0. Dies bedeutet, dass das zweite Kanalelement eine Kanalverzweigung mit dem dritten Kanalelement aufweist. Die Zeile 715 stellt eine Kanalverzweigung zwischen dem dritten Kanalelement und dem zweiten Kanalelement dar. Die Zeile 720 stellt eine Kanalverzweigung des vierten Kanalelementes mit dem fünften Kanalelement dar. In Zeile 725 ist eine Kanalverzweigung des fünften Kanalelementes mit dem vierten Kanalelement dargestellt. Die Zeile 730 gibt wieder, dass das sechste Kanalelement keine Kanalverzweigung aufweist.

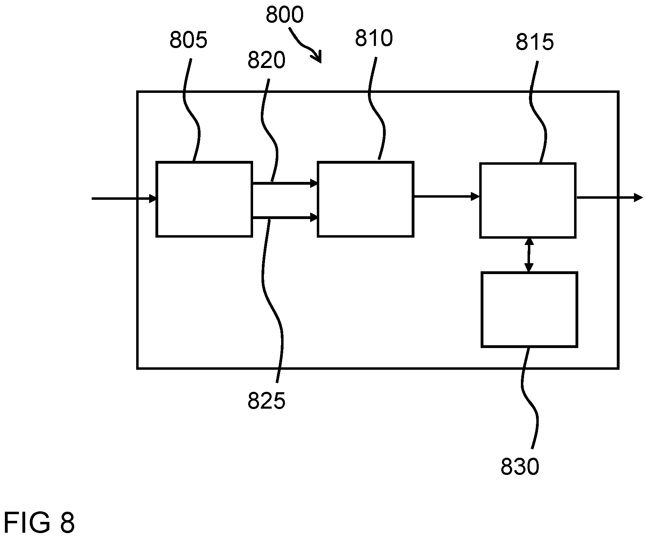

Figur 8 zeigt ein Blockschaltbild einer Vorrichtung 800.

Die Vorrichtung 800 weist eine Beaufschlagungseinrichtung 805, eine Einleseeinrichtung 810 und eine Erkennungseinrichtung 815 auf. Die Beaufschlagungseinrichtung 805 ist ausgebildet, um das erste Kanalelement mit einem Prüfdruck zu beaufschlagen. Die Einleseeinrichtung 810 ist ausgebildet, um ein Drucksignal 820 einzulesen, wobei das Drucksignal 820 einen Druck in dem zweiten Kanalelement repräsentiert. Die Erkennungseinrichtung 815 ist ausgebildet, um die Verbindung zwischen dem ersten und dem zumindest zweiten Kanalelement zu erkennen, wenn der Druck in einem vorbestimmten Verhältnis zum Prüfdruck und/oder über einem Schwellwert liegt.

Gemäß einem Ausführungsbeispiel ist die Einleseeinrichtung 810 ausgebildet, um ein weiteres Drucksignal 825 einzulesen, um eine Verbindung zwischen dem ersten und zumindest einem dritten in dem Spülgerät befindlichen Kanalelement zu ermitteln. Das weitere Drucksignal 825 repräsentiert einen weiteren Druck in dem dritten Kanalelement. Die Erkennungseinrichtung 815 ist ferner ausgebildet, um die Verbindung zwischen dem ersten und dem zumindest dritten Kanalelement zu erkennen, wenn der weitere Druck in einem vorbestimmten Verhältnis zum Prüfdruck und/oder über einem Schwellwert liegt.

Gemäß einem Ausführungsbeispiel ist die Beaufschlagungseinrichtung 805 ausgebildet, um das zweite Kanalelement mit einem zweiten Prüfdruck zu beaufschlagen, um eine Verbindung zwischen dem zweiten und zumindest dem dritten in dem Spülgerät befindlichen Kanalelement zu ermitteln. Ansprechend darauf wird in der Erkennungseinrichtung 815 die Verbindung zwischen dem zweiten und dem zumindest dritten Kanalelement erkannt, wenn der weitere Druck in einem vorbestimmten Verhältnis zum weiteren Prüfdruck und/oder über einem weiteren Schwellwert liegt.

Gemäß einem weiteren Ausführungsbeispiel weist die Vorrichtung 800 eine Speichereinrichtung 830 auf, die ausgebildet ist, um eine Information über das Vorhandensein der Verbindung in einer Tabelle zu speichern. Dabei repräsentieren die Zeilen und die Spalten je eines der Kanalelemente.

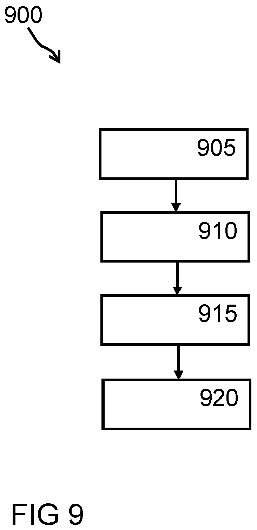

Figur 9 zeigt ein Ablaufdiagramm eines Ausführungsbeispiels eines Verfahrens 900 zum Ermitteln einer Verbindung zwischen einem ersten und zumindest einem zweiten in einem Spülgerät befindlichen Kanalelement. Das Verfahren 900 weist einen Schritt 905 des Beaufschlagens des ersten Kanalelements mit einem Prüfdruck auf, sowie einen Schritt 910 des Einlesens eines Drucksignals, das einen Druck in dem zweiten Kanalelement repräsentiert und einen Schritt 915 des Erkennens der Verbindung zwischen dem ersten und dem zumindest zweiten Kanalelement, wenn der Druck in einem vorbestimmten Verhältnis zum Prüfdruck und/oder über einem Schwellwert liegt. Im Schritt 905 des Beaufschlagens wird beispielhaft Druckluft und/oder eine Flüssigkeit in das erste Kanalelement gepumpt, um den Prüfdruck aufzubauen. Ferner wird im Schritt 905 des Beaufschlagens der Prüfdruck in ein medizinisches Gerät als Kanalelement, insbesondere in ein Endoskopelement beaufschlagt. Im Schritt 910 des Einlesens wird dann ein Druck in einem zweiten medizinischen Gerät als zweites Kanalelement, insbesondere in einem zweiten Endoskopelement eingelesen. Im Schritt 905 des Beaufschlagens wird das Kanalelement beispielhaft unter Verwendung eines 3/2-Wege-Ventils mit dem Prüfdruck beaufschlagt. Gemäß einem Ausführungsbeispiel wird im Schritt 905 des Beaufschlagens das Kanalelement durch einen Druckanschluss eines Pulsationsmoduls mit dem Prüfdruck beaufschlagt. Im Schritt 910 des Einlesens wird dann das Drucksignal von einem Drucksensor des Pulsationsmoduls eingelesen, insbesondere wobei der Drucksensor und der Druckanschluss in einem gemeinsamen Pulsationsmodulgehäuse angeordnet sind.

Gemäß einem Ausführungsbeispiel ist das Verfahren 900 zum Ermitteln einer Verbindung zwischen dem ersten und zumindest einem dritten in dem Spülgerät befindlichen Kanalelement ausgebildet. Dazu wird im Schritt 910 des Einlesens ein weiteres Drucksignal eingelesen, das einen weiteren Druck in dem dritten Kanalelement repräsentiert. Im Schritt 915 des Erkennens wird dann die Verbindung zwischen dem ersten und dem zumindest dritten Kanalelement erkannt, wenn der weitere Druck in einem vorbestimmten Verhältnis zum Prüfdruck und/oder über einem Schwellwert liegt.

Gemäß einem weiteren Ausführungsbeispiel ist das Verfahren 900 zum Ermitteln einer Verbindung zwischen dem zweiten und zumindest dem dritten in dem Spülgerät befindlichen Kanalelement ausgebildet. Dazu wird im Schritt 915 des Beaufschlagens das zweite Kanalelement mit einem zweiten Prüfdruck beaufschlagt. Im Schritt 915 des Erkennens wird dann die Verbindung zwischen dem zweiten und dem zumindest dritten Kanalelement erkannt, wenn der weitere Druck in einem vorbestimmten Verhältnis zum weiteren Prüfdruck und/oder über einem weiteren Schwellwert liegt.

Gemäß einem Ausführungsbeispiel wird vor dem Schritt 905 des Beaufschlagens des zweiten Kanalelements mit dem zweiten Prüfdruck der Prüfdruck aus dem ersten Kanalelement abgelassen und/oder zumindest reduziert.

Das Verfahren 900 weist gemäß einem Ausführungsbeispiel einen Schritt 920 des Speicherns einer Information über das Vorhandensein der Verbindung in einer Tabelle auf, insbesondere wobei die Zeilen und die Spalten je eines der Kanalelemente repräsentieren.

Die Schritte des Beaufschlagens 905, des Einlesens 910 und/oder des Erkennens 915 werden ausgeführt, wenn eine Umwälzpumpe zur Umwälzung einer Behandlungsflotte eingeschaltet oder ausgeschaltet ist.

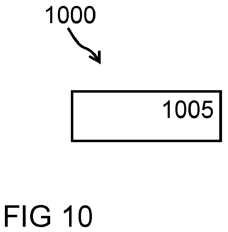

Figur 10 zeigt ein Ablaufdiagramm eines Ausführungsbeispiels eines Verfahrens 1000 zum Reinigen eines Kanalelements. Das Verfahren 1000 umfasst einen Schritt 1005 des Durchleitens einer Behandlungsflotte durch das erste und/oder zweite Kanalelement ansprechend auf die in Figur 9 durch ein Verfahren erkannte Verbindung.

Alle genannten Merkmale, auch die den Zeichnungen allein zu entnehmenden sowie auch einzelne Merkmale, die in Kombination mitanderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein.

## Patentansprüche

1. Verfahren (900) zum Ermitteln einer Verbindung zwischen einem ersten (300) und zumindest einem zweiten (305) in einem Spülgerät (100) befindlichen Kanalelement, wobei das Verfahren (900) die folgenden Schritte aufweist:
- Beaufschlagen (905) des ersten Kanalelements (300) mit einem Prüfdruck;
- Einlesen (910) eines Drucksignals (820), das einen Druck in dem zweiten Kanalelement (305) repräsentiert; und
- Erkennen (915) der Verbindung zwischen dem ersten (300) und dem zumindest zweiten Kanalelement (305), wenn der Druck in einem vorbestimmten Verhältnis zum Prüfdruck und/oder über einem Schwellwert liegt.

2. Verfahren (900) gemäß Anspruch 1, zum Ermitteln einer Verbindung zwischen dem ersten (300) und zumindest einem dritten (310) in dem Spülgerät (100) befindlichen Kanalelement, wobei im Schritt (910) des Einlesens ein weiteres Drucksignal (825) eingelesen wird, das einen weiteren Druck in dem dritten Kanalelement (310) repräsentiert, wobei im Schritt (915) des Erkennens die Verbindung zwischen dem ersten (300) und dem zumindest dritten Kanalelement (310) erkannt wird, wenn der weitere Druck in einem vorbestimmten Verhältnis zum Prüfdruck und/oder über einem Schwellwert liegt.

3. Verfahren (900) gemäß Anspruch 2, zum Ermitteln einer Verbindung zwischen dem zweiten (305) und zumindest dem dritten (310) in dem Spülgerät (100) befindlichen Kanalelement, wobei im Schritt (905) des Beaufschlagens das zweite Kanalelement (305) mit einem zweiten Prüfdruck beaufschlagt wird, wobei im Schritt (915) des Erkennens die Verbindung zwischen dem zweiten (305) und dem zumindest dritten Kanalelement (310) erkannt wird, wenn der weitere Druck in einem vorbestimmten Verhältnis zum weiteren Prüfdruck und/oder über einem weiteren Schwellwert liegt.

4. Verfahren (900) gemäß Anspruch 3, wobei vor dem Schritt (905) des Beaufschlagens des zweiten Kanalelements (305) mit dem zweiten Prüfdruck der Prüfdruck aus dem ersten Kanalelement (300) abgelassen und/oder zumindest reduziert wird.

5. Verfahren (900) gemäß einem der vorherigen Ansprüche, mit einem Schritt (920) des Speicherns einer Information über das Vorhandensein der Verbindung in einer Tabelle, insbesondere wobei die Zeilen (705, 710, 715, 720, 725, 730) und die Spalten je eines der Kanalelemente (300, 305, 310, 315, 320, 325) repräsentieren.

6. Verfahren (900) gemäß einem der vorherigen Ansprüche, wobei im Schritt (905) des Beaufschlagens Druckluft und/oder eine Flüssigkeit in das erste Kanalelement (300) gepumpt wird, um den Prüfdruck aufzubauen.

7. Verfahren (900) gemäß einem der vorherigen Ansprüche, wobei im Schritt (905) des Beaufschlagens der Prüfdruck in ein medizinisches Gerät als Kanalelement (300), insbesondere in ein Endoskopelement beaufschlagt wird und/oder wobei im Schritt (910) des Einlesens ein Druck in einem zweiten medizinischen Gerät als zweiten Kanalelement (305), insbesondere in einem zweiten Endoskopelement eingelesen wird.

8. Verfahren (900) gemäß einem der vorherigen Ansprüche, wobei im Schritt (905) des Beaufschlagens das Kanalelement (300) unter Verwendung eines 3/2-Wege-Ventils mit dem Prüfdruck beaufschlagt wird.

9. Verfahren (900) gemäß einem der vorherigen Ansprüche, wobei im Schritt (905) des Beaufschlagens das Kanalelement (300) durch einen Druckanschluss eines Pulsationsmoduls (150) mit dem Prüfdruck beaufschlagt wird und wobei im Schritt (910) des Einlesens das Drucksignal (820) von einem Drucksensor (210) des Pulsationsmoduls (150) eingelesen wird, insbesondere wobei der Drucksensor (210) und der Druckanschluss in einem gemeinsamen Pulsationsmodulgehäuse angeordnet sind.

10. Verfahren (900) gemäß einem der vorherigen Ansprüche, wobei die Schritte des Beaufschlagens (905), des Einlesens (910) und/oder des Erkennens (915) ausgeführt werden, wenn eine Umwälzpumpe (115) zur Umwälzung einer Behandlungsflotte eingeschaltet oder ausgeschaltet ist.

11. Verfahren (1000) zum Reinigen eines Kanalelements, mit einem Schritt (1005) des Durchleitens einer Behandlungsflotte durch das erste (300) und/oder zweite Kanalelement (305) ansprechend auf die in einem Verfahren (900) gemäß einem der Ansprüche 1 bis 10 erkannte Verbindung.

12. Vorrichtung (800), die ausgebildet ist, um die Schritte (905, 910, 915, 920; 1005) eines der Verfahren (900; 1000) gemäß einem der vorherigen Ansprüche 1 bis 10 oder 11 in entsprechenden Einheiten (805, 810, 815, 830) auszuführen und/oder anzusteuern.

13. Spülgerät (100) mit einer Vorrichtung (800) gemäß Anspruch 12, einer Spülkammer (105) und zumindest einem Spülkorb (110).

14. Computerprogrammprodukt mit Programmcode zur Durchführung des Verfahrens (900) nach einem der vorherigen Ansprüche, wenn das Computerprogrammprodukt auf einer Vorrichtung (800) ausgeführt wird.
